# EUROPÄISCHE PATENTANMELDUNG

(11) **EP 4 745 546 A1**
(43) Veröffentlichungstag der Anmeldung: **20.05.2026**
(21) Anmeldenummer: 24214003.6
(22) Anmeldetag: 19.11.2024
(51) Int. Cl.: G01L 1/04, A61B 5/22, G01L 1/22, G01L 5/22

(54) **SYSTEM FÜR EIN RUDER ZUR BESTIMMUNG DER RUDERKRAFT WÄHREND EINES RUDERSCHLAGS**

(71) Anmelder: Mandanis angewandte Mechanik GmbH, 6010 Kriens (CH)
(72) Erfinder: MANDANIS, Georges, 6010 Kriens (CH)
(74) Vertreter: Piticco, Lorena

(57) **Zusammenfassung**

Ein System für ein Ruder zur Bestimmung der Ruderkraft während eines Ruderschlags eines Ruderers im Wasser hat eine Messaufnahme (3) mit einem ruderschaftseitigen Aufnahmebereich (12) für eine Befestigung mit einem Ruderschaft (1) eines Ruders und mit einem rudergriffseitigen Aufnahmebereich (13) für eine Befestigung mit einem Rudergriff (9) diesen Ruders. Dabei ist eine Platte (5, 15) zwischen den Aufnahmebereichen vorgesehen, auf deren beiden Seiten jeweils ein Dehnungsmessstreifen (4, 14) nahe des ruderschaftseitigen Aufnahmebereichs (12) oder nahe des rudergriffseitigen Aufnahmebereichs (13) aufgebracht ist, aus dessen Signal sich die Ruderkraft bestimmen lässt, da bei einer Kraftausübung (101) auf den rudergriffseitigen Aufnahmebereich (13) senkrecht zur Ebene der Platte (5, 15) die Platte eine S-Form annimmt.

## Beschreibung

### TECHNISCHES GEBIET

Die vorliegende Erfindung betrifft ein System für ein Ruder zur Bestimmung der Ruderkraft während eines Ruderschlags eines Ruderers im Wasser und ein Ruder mit einem solchen System.

### STAND DER TECHNIK

Aus der EP 3 187 849 ist ein System und Verfahren der eingangs genannten Art bekannt. Dieses System ermöglicht beispielsweise die Überwachung der Ruderschläge beim Training oder beim Wettkampf des Ruderers durch den Ruderer selber oder durch eine Drittperson, wie beispielsweise einen Trainer. Es erlaubt eine detaillierte Analyse des Ruderschlags, mit dem Ziel, diesen zu verbessern. Es erlaubt auch Vergleiche mit gespeicherten Werten, wie mit denen einer früheren Messreihe, beispielsweise eines früheren Trainings oder Rennen oder erlaubt einen Vergleich mit anderen Ruderern, beispielsweise einem Teamkollegen oder einem Weltmeister. Diese Vergleiche helfen dem Ruderer oder dem Trainer, die Leistung des einzelnen Ruderers besser einschätzen zu können und somit ein gezieltes Training zusammenzustellen. Auch für Freizeitruderer ist das System geeignet, da es die eigene Leistung und somit die Entwicklung erkennbar macht.

### DARSTELLUNG DER ERFINDUNG

Das System nach der EP 3 187 849 erfordert eine Unterteilung des Ruderschafts. Das System ist dann an der Trennstelle eingesetzt. Es ist daher unter anderem eine Aufgabe der Erfindung, ein System zur Verfügung zu stellen, bei welchem die Montage der Sensoren vereinfacht ist, und welches zuverlässiger, günstiger oder einfacher in der Bedienung ist.

Diese Aufgabe wird durch ein System mit den Merkmalen des Anspruchs 1 gelöst.

Ein System für ein Ruder zur Bestimmung der Ruderkraft während eines Ruderschlags eines Ruderers im Wasser weist eine Messaufnahme mit einem ruderschaftseitigen Aufnahmebereich für eine Befestigung mit einem Ruderschaft eines Ruders und mit einem rudergriffseitigen Aufnahmebereich für eine Befestigung mit einem Rudergriff diesen Ruders auf. Mindestens ein in der Messaufnahme angeordneter Dehnungsmessstreifen gestattet es, aus dessen Signal eine Ruderkraft zu bestimmen, wobei eine mit dem oder den Dehnungsmessstreifen verbundene Auswerteschaltung eingesetzt wird.

Wesentlich ist, dass die Längsachse der Befestigung der Messaufnahme an dem Ruderschaft mit der Längsachse der Befestigung der Messaufnahme an dem Rudergriff ohne Belastung zusammenfällt, wobei dann die Messaufnahme mindestens eine Platte umfasst, die sich zwischen dem ruderschaftseitigen Aufnahmebereich und dem rudergriffseitigen Aufnahmebereich erstreckt, so dass bei einer Kraftausübung auf den rudergriffseitigen Aufnahmebereich senkrecht zur Ebene der Platte die Platte eine S-Form annimmt. Bei der Erfindung sind dann zwei Dehnungsmessstreifen vorgesehen, die nahe des ruderschaftseitigen Aufnahmebereichs oder nahe des rudergriffseitigen Aufnahmebereichs auf beiden Seiten der besagten Platte aufgebracht sind, wobei die Auswerteschaltung ausgestaltet ist, um aus der Dehnung und Stauchung der beiden Dehnungsmessstreifen die auf den Handgriff ausgeübte Kraft zu berechnen.

Ein Vorteil dieser Anordnung liegt darin, dass bei einem bestehenden Ruder lediglich der Handgriff durch einen Handgriff mit dem hier offenbarten System zu ersetzen ist, da an und in diesem alle Bauteile für die Messung der Kraft und der Rotationsbewegung des Ruders integrierbar sind. Als weiterer Input für die Auswertung ist nur die Entfernung von der Messwertaufnahme zur Dolle festzustellen und bei der Auswertung einzusetzen.

Statt der Bestimmung eines Biegemoments an einem Ruder während eines Ruderschlags eines Ruderers im Wasser wird hier direkt die Kraft am Handgriff über zwei Dehnungsmessstreifen (hier auch kurz als DMS bezeichnet) gemessen, aus deren Signalen sich das damit erzeugte Moment bestimmen lässt. Die Schlagzahl des Ruderers, d.h. die Anzahl Ruderschläge über die Zeit, lässt sich ebenfalls mit integrierten Sensoren von dem System ermitteln.

Vorteilhafterweise sind zwei Platten vorgesehen, die in einem radialen Abstand von der Längsachse des ruderschaftseitigen Aufnahmebereichs zur Befestigung der Messaufnahme in Richtung der Kraftausübung parallel zueinander vorgesehen sind. Das ermöglicht eine Anordnung des Messsystems dicht am Handgriff selber aber im Bereich des Ruderschaftendes. Wenn der Abstand von der Längsachse der beiden Platten gleich ist, kann das Ruderschaftende vorteilhafterweise in einer Hülse zwischen den Platten angeordnet sein, um eine längere Führung zu ermöglichen, insbesondere über die gesamte Messaufnahme bis in den Bereich des Handgriffs. Dafür kann ein Innenteil vorgesehen sein, welches in der Messaufnahme zwischen den Platten angeordnet ist und insbesondere mit einem Gewinde in einer Hülse des Handgriffs jenseits der Platten montiert sein kann. Dabei kann in Richtung Handgriff eine sich als Kegelstumpf verjüngende Hülse die Führung bilden.

Bei einem vorteilhaften System ist zudem ein mikroelektromechanisches System zur Feststellung der Rotationsbewegung vorgesehen, um dann über eine Auswerteschaltung mit einem aus der gemessenen Kraft ermitteltem Drehmoment über einen gespeicherten Abstandswert der Messaufnahme von der Dolle die Ruderleistung zu ermitteln.

Dabei können vorteilhafterweise die beiden vom linken und rechten Ruder ermittelten Messwerte an eine externe Auswerteschaltung übermittelt werden, die dann die jeweiligen Leistungen berechnet. Eine solche Auswerteschaltung kann als Software in einem Tablet oder Smartphone ausgestaltet sein.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine Seitenansicht eines Ruders mit einem System nach einem Ausführungsbeispiel der Erfindung;
- Fig. 2: eine perspektivische Ansicht des Griffs des Ruders nach Fig. 1 mit dem System ohne ein abdeckendes Gehäuse und ohne Innenteil schräg von oben;
- Fig. 3: eine andere perspektivische Ansicht des Griffs ähnlich zu Fig. 2 schräg von unten;
- Fig. 4: eine Seitenansicht eines Innenteil zur Montage des Ruderschaftes;
- Fig. 5: eine Seitenansicht eines Teil eines Griffs in Relation zum Innenteil nach Fig. 4;
- Fig. 6: eine Seitenansicht des Griffs in Ruheposition und eine Seitenansicht nach einem Zug auf den Handgriff in Zugrichtung; und
- Fig. 7: ein Diagramm der gemessenen Kraft über die Zeit für ein an Backbord und ein an Steuerbord in einem Ruderhandgriff angeordnetes System nach einem Ausführungsbeispiel.

### BESCHREIBUNG BEVORZUGTER AUSFÜHRUNGSFORMEN

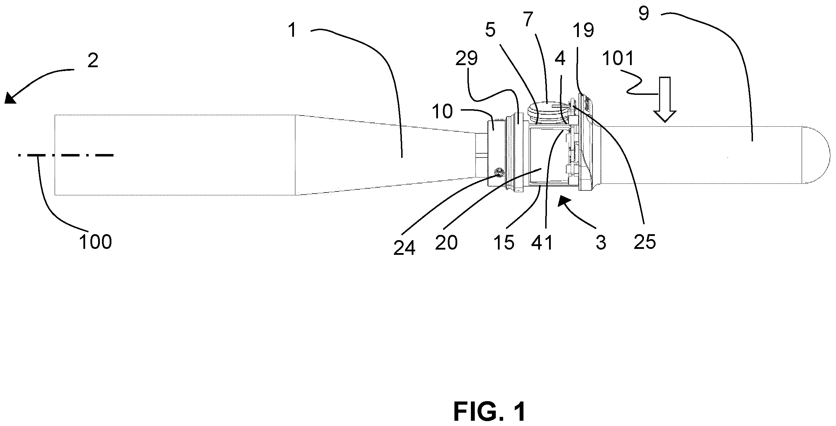

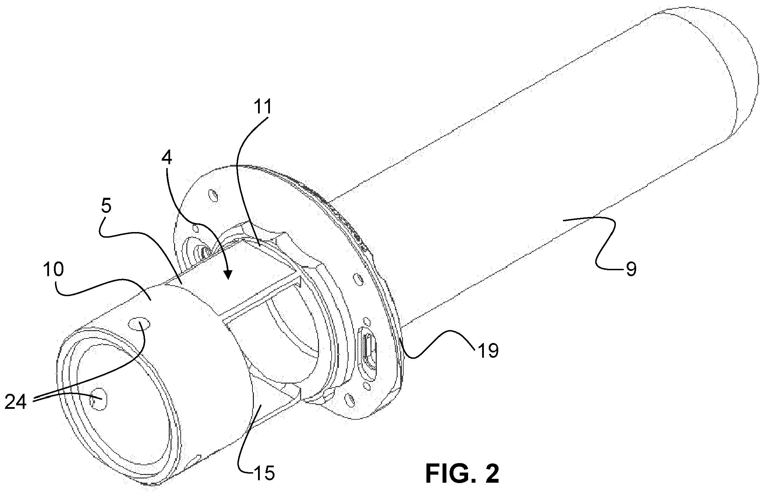

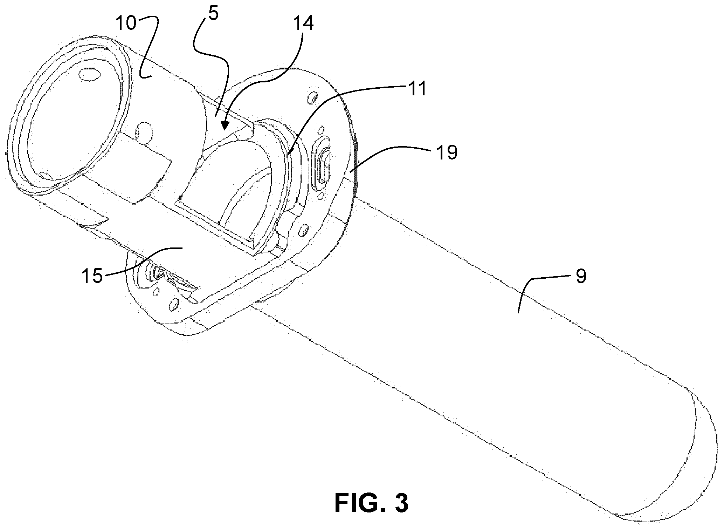

Die Fig. 1, 2 und 3 sind zusammen zu betrachten. Dabei zeigt Fig. 1 eine Seitenansicht eines Ruders mit einem System nach einem Ausführungsbeispiel der Erfindung. Fig. 2 zeigt eine perspektivische Ansicht des Rudergriffs 9 aus Fig. 1 mit dem System ohne Darstellung von elektronischen Komponenten, ohne ein abdeckendes Gehäuse und ohne Innenteil 20 schräg von oben. Fig. 3 zeigt eine andere perspektivische Ansicht des Rudergriffs 9 ähnlich zu Fig. 2 schräg von unten.

Die Fig. 1 zeigt eine Seitenansicht eines erfindungsgemässen Systems mit einem Ruder mit einem Ruderschaft 1, welcher in einem hier nicht dargestellten Ruderblatt 2 endet. Das Ruder weist auf der dem Ruderblatt gegenüberliegenden Seite weiterhin einen Rudergriff 9 auf. Zwischen dem Ruderschaft 1 und dem Rudergriff 9 ist eine Messaufnahme 3 angeordnet, welche den Ruderschaft 1 mit dem Handgriff 9 verbindet.

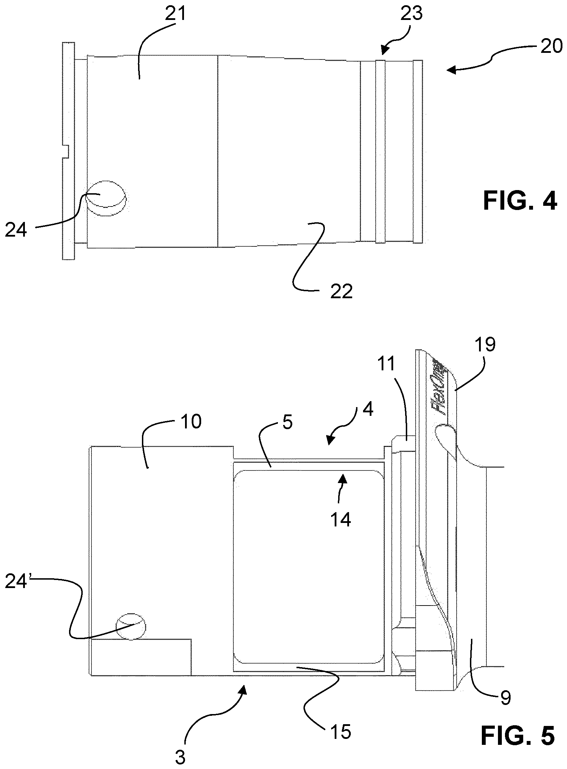

Die Fig. 1 zeigt hier ein zentrales Innenteil 20, beispielsweise in Hülsenform, welches an zwei gegenüberliegenden Seiten von einer oberen Klinge 5 und einer unteren Klinge 15 umgeben ist. Dabei bedeutet "oben" entgegen der Zugrichtung 101 und "unten" in Zugrichtung. Die beiden Klingen 5 und 15 sind dünne Platten. Die Klingen 5 und 15 sind links, also an der Ruderschaftseite der Messaufnahme 3, jeweils mit dieser verbunden. Die Klingen 15 sind rechts, also an der Handgriffseite der Messaufnahme 3, ebenfalls jeweils mit dieser verbunden. Die dünnen Platten 5, 15 sind ohne Belastung parallel zueinander angeordnet und senkrecht zur Zugrichtung 101 ausgerichtet. Jede der Platten 5, 15 reagiert wie ein beidseitig eingespannter Balken. Die Messaufnahme 3 weist an beiden Enden der dünnen Platten 5 und 15 jeweils eine Hülse zur Befestigung an dem Ruderschaft 1 bzw. an dem Handgriff 9 auf. Vorteilhafterweise ist radial ausserhalb der oberen Klinge 5 eine Verarbeitungseinheit und eine Energiequelle, beispielsweise ein Akkumulator oder eine Batterie 7, oberhalb von deren Aussenseite an einem PCB 25 angeordnet. Die Verarbeitungseinheit kann auch im PCB 25 enthalten sein. Sofern das später in Fig. 4 genauer dargestellte Innenteil 20 keinen Kegelstumpfabschnitt 22 aufweist und somit der Endbereich der Hülse 23 bereits am Ende des Aussengewindes 21 endet, könnte sowohl das PCB 25 als auch die Batterie 7 im Innern des hohlen Ruderschafts 1 oder im Handgriff 9 angeordnet sein. Der PCB 25ist hier an dem als Flansch ausgebildeten Anschlag 19 des Handgriffs 9 befestigt. Mit dem Bezugszeichen 29 ist die als Dichtungsring 29 auf dem Aussenmantel der ruderschaftseitigen Hülse 10 angeordnete Dichtung bezeichnet. Üblicherweise ist diese Dichtung mit einem in den Fig. nicht dargestellten Gehäuse um den Bereich der Messaufnahme 3 verbunden. Das Gehäuse kann dann auf der anderen Seite an dem Flansch 19 befestigt sein. Damit kann die gesamte Messaufnahme dicht positioniert werden..

Sowohl die ruderschaftseitige Hülse 10 als auch die rudergriffseitige Hülse 11 und die zwischen diesen bestehende stegförmige Verbindung über die Klingen 5 und 15 können begriffsmässig dem Rudergriff 9 zugeordnet werden, da sie eine einstückige Verbindung bilden.

Auf der oberen Klinge 5 ist an der von der Achse 100 und damit von der unteren Klinge 15 wegweisenden Fläche ein äusserer Dehnungsmesstreifen 4 befestigt und auf der oberen Klinge 5 ist ebenfalls an der zu der Achse 100 und damit zu der unteren Klinge 15 hinweisenden Fläche ein innerer Dehnungsmesstreifen 14 befestigt. Der innere Dehnungsmesstreifen 14 ist damit in der Nähe aber in einem Abstand ausserhalb von dem Innenteil 20 angeordnet. Beide Klingen 5 und 15 sind - ohne Belastung des Handgriffs 9 - vorteilhafterweise in einem selben Abstand von der zentralen Längsachse des Handgriffs 9 angeordnet.

Die Fig. 4 zeigt eine Seitenansicht eines Innenteils 20 zur Montage des Ruderschaftes 1, während Fig. 5 eine Seitenansicht eines zeichnerisch direkt darunter angeordneten Teil eines Griffs 9 in Relation zu diesem Innenteil 20 nach Fig. 4 umfasst. Im zusammengebauten Zustand ist das Innenteil 20 in der im oder vor dem Handgriff 9 angeordneten Messaufnahme 3 eingeschoben und fixiert. Dafür weist das Innenteil 20 in seinem hülsenförmigen ruderschaftseitigen Ende über ein Aussengewinde 21, dem gegenüber ein in der ruderschaftseitigen Hülse 10 vorgesehenes Innengewinde vorgesehen ist, so dass das Innenteil eingeschoben verschraubt werden kann. Im Prinzip sind auch andere Verriegelungsverfahren einsetzbar. Der Vorteil des Innenteils 20 als Hülse ist die längere Führung für den Ruderschaft 1, der durch die Hülse hindurch, also innerhalb des Bereichs mit dem Aussengewinde 21 und dem kegelstumpfförmigen Hülsenabschnitt 22 zwischen den Klingen 5 und 15 bis in den Endbereich 23 des Innenteils 20 reicht.

Beide Elemente, das Innenteil 20 und die Messaufnahme 3 des Handgriffs 9 sind Aluminiumteile, welche die tragende Struktur darstellen. Nur die dünnen Platten 5 und 15 verbinden diese ruderschaftseitige Hülse 10 mit dem Handgriff 9. Dabei weist das Innenteil 20 an den Abschnitt 21 mit dem Aussengewinde anschliessend einen sich leicht verjüngenden Kegelstumpfabschnitt 22 auf, so dass sich schon ergibt, dass sich die rudergriffseitige Hülse 11 in Richtung der Zugrichtung 101 des an einem Boot befestigten Ruders bewegen kann. Mit den Bezugszeichen 4 und 14 sind die Positionen markiert, an denen in Stegrichtung der oberen Klinge 5 die DMS nach aussen und nach innen gerichtet auf der Klinge 5 befestigt sind. Alternativ können die DMS auch an der unteren Klinge 15 in gleicher Weise nach aussen und nach innen gerichtet auf der Klinge 15 befestigt sein.

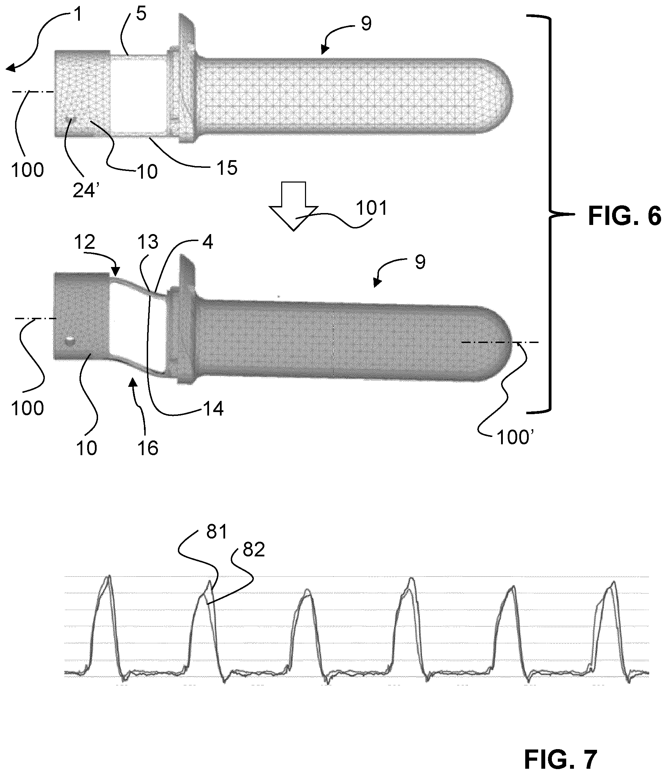

Die Fig. 6 zeigt eine Seitenansicht des Handgriffs 9 in Ruheposition und nach einem Zug auf den Handgriff 9 in Zugrichtung 101. Weitere Elemente des Ruders sind nicht dargestellt. Diese sind mit der ruderschaftseitigen Hülse 10 verbunden. Diese ist mit dem Ruderschaft um die Längsachse 100 des Ruders (ohne Belastung) verbunden. Diese Längsachse 100 ist auch die Längsachse 100 des Handgriffs 9 in der Ruhestellung.

Der Hülsenteil 10, der über die Klingen 5, 15 mit dem Handgriff 9 verbunden ist, und der Innenteil 20 (in Fig. 6 nicht dargestellt), der über das Aussengewinde 21 auf dem Innenteil 20 mit einem entsprechenden Innengewindes des Hülsenteils 10 fest verbunden ist, sind die einzigen Elemente, die mit dem Ruderschaft 1 verbunden sind; ansonsten steht der Innenteil 20 nicht in Kontakt mit dem Griff. Der Ruderschaft 1 wird spielfrei in den Innenteil 20 geführt und mit drei Schrauben (nicht dargestellt) in entsprechenden Fixierungslöchern 24' in der Messaufnahme 3 und Durchgangslöchern 24 im Innenteil 20 fixiert. Dieses Design sichert eine feste zwangslose Verbindung zwischen dem Handgriff 9 und dem Ruderschaft 1 mit einem langen Führungsweg über die Länge des Innenteils 20, welche während dem Rudern nicht gelockert werden kann.

Die Messelemente sind zwei Dehnmessstreifen (DMS) 4 und 14, welche auf der oberen Klinge 5 des Handgriffes 9 einander gegenüberliegend aufgeklebt sind. Die beiden DMS 4 und 14 müssen dabei genau übereinander und symmetrisch gegenüber der vertikalen Symmetrieebene liegen.

Die Dehnmessstreifen messen Kraft des Ruderers am Griff 9. Wenn diese gemessene Kraft mit dem Abstand zwischen Handgriff und Dolle multipliziert wird, dann ergibt sich das entsprechende Moment.

Durch die Kraftbelastung in Zugrichtung 101 verformen sich die obere Klinge 5 und die untere Klinge 15 S-förmig. Diese S-förmige Verformung 16 tritt an den Einspannstellen der Klingen 5 oder 15 auf; damit bilden der ruderschaftseitige Aufnahmebereich 12 bzw. der rudergriffseitige Aufnahmebereich 13 die geeigneten Bereiche für das Befestigen, insbesondere Aufkleben der Dehnmessstreifen 4 und 14 auf gegenüberliegenden Seiten der Klingen 5 oder 15. Mit anderen Worten, es bestehen vier Möglichkeiten für eine Montage der DMS. Es wird in der Regel nur eine eingesetzt. Es ist aber auch möglich aus Redundanzgründen und zur Erhöhung der Messgenauigkeit durch zwei unabhängige Messungen auch zwei Paare von DMS einzusetzen, beispielsweise an beiden Klingen 5 und 15.

Der Handgriff 9 wird durch den Zug des Ruderers in Richtung 101 bewegt, also senkrecht zur Längsachse 100 des Ruders (ohne Belastung). Diese Bewegung kann beispielsweise einen Hub von maximal ca. 0.1 Millimeter haben. Dies ist im Ansatz keine Schwenkbewegung des Handgriffes 9 sondern eine translatorische oder laterale Bewegung, die zu einer Verschiebung (Offset) der Längsachse 100' des Handgriffs 9 unter Belastung führt. Der Zug des Ruderers führt zu einer Griffdeformation durch Verbiegung der Klingen 5 und 15 an den Einspannstellen, was zu der genannten S-Form 16 führt.

Die Verarbeitungseinheit 50 ist mit den beiden DMS 4 und 14 verbunden, mit denen die Differenz der Dehnungen zwischen der oberen und der unteren Seite der oberen Klinge 5 gemessen wird. Wie schon oben ausgeführt, kann diese Messung auch an der unteren Klinge 15 und an dem ruderschaftseitiger Aufnahmebereich 12 vorgenommen werden, wenn auch die Messung durch DMS an dem handgriffseitigen Aufnahmebereich 13 bevorzugt ist. Diese Differenz ist am grössten an den Einspannstellen links und rechts und null in der Mitte zwischen den beiden Einspannstellen. Dort ist ein Wendepunkt, also keine Krümmung und keine Dehnungsdifferenz. Aus diesem Grund müssen die DMS so nah wie möglich an einer oder mehreren der Einspannstellen liegen.

Diese Messung reagiert nur auf die Kraft. Das Moment, welches durch die Kraft erzeugt wird, wird durch eine Zugkraft im Querschnitt der oberen Klinge 5 und durch eine Druckkraft im Querschnitt der unteren Klinge 15 übernommen. Beide DMS 4 und 14 an einer Klinge 5 oder 15 produzieren für diese Belastung das gleiche Signal, also die Differenz bleibt null. Diese DMS-Anordnung misst also tatsächlich nur die Kraft und kein Moment.

Die Erfindung beruht inter alia darauf, dass für die Bestimmung der Ruderleistung eine Kraftmessung an einer doppelt eingespannten flexiblen Klinge möglich ist. Vorteilhafterweise ist eine zweite Klinge auf der gegenüberliegenden Seite der Längsachse des Handgriffs vorgesehen, um das freie Ende des Ruderschaftes 1 in dem Innenteil 20 bis zum Handgriffbereich zu führen. Vorteilhafterweise ist ein weiterer Sensor zur Feststellung der Rotationsgeschwindigkeit des Ruders vorgesehen, wobei somit zwei Messwerte ermittelt werden können, nämlich Kraft und Rotationsgeschwindigkeit; beides über die Zeit gemessen, um die gewünschte Grösse der Ruderleistung festzustellen. Dabei ist für die Feststellung des Wertes wesentlich, dass man über Zeitintervalle eines Ruderschlags arbeitet und sich nicht für die Kraft allein oder das Moment interessiert. Denn Moment und Rotationsgeschwindigkeit werden dafür parallel über die Zeit am gleichen Ort, nämlich in und nahe der Messbrücke der beiden DMS 4 und 14 gemessen. Dafür muss das Drehmoment und die Rotationsgeschwindigkeit an der Dolle erfasst werden, wobei hier durch die Messbrücke in einem Abstand von der Dolle die gemessene Kraft mit dem besagten Abstand multipliziert wird, um das Drehmoment zu bestimmen.

Vorzugsweise ist der Handgriff im Wesentlichen zylindrisch mit einer Mittelachse 100 ausgebildet. Da der Ruderschaft 1 im Allgemeinen einen im Wesentlichen kreisrunden Querschnitt aufweist, eignet sich eine zylindrische Form als Verbindungselement mit zwei flachen gegenüberliegenden Klingen 5 und 15. Alternativ können Messhülsen mit ovalen oder vieleckigen Querschnitten, wie zum Beispiel rechteckigen, quadratischen oder sechseckigen Querschnitten verwendet werden, so lange die beiden genannten Klingen 5 und 15 so vorgesehen werden, dass der Zug des Ruderers auf den Handgriff in Richtung 101 senkrecht zur Anordnung der Klingen 5 und 15 ausgeführt wird. An diesen Auflageflächen können aufgrund der Richtung der Krafteinleitung am Ruderblatt, d.h. im Wesentlichen senkrecht auf das Ruderblatt, die grössten Dehnungen gemessen werden.

Die Fig. 7 zeigt ein Diagramm der gemessenen Kraft 81 oder 82 über die Zeit für ein an Backbord und ein an Steuerbord in einem Ruderhandgriff 9 angeordnetes System nach einem Ausführungsbeispiel der Erfindung. Vorteilhafterweise werden dabei die beiden vom linken und rechten Ruder ermittelten Messwerte an eine externe Auswerteschaltung übermittelt, die dann die jeweiligen Leistungen berechnet. Eine solche Auswerteschaltung kann als Software in einem Tablet oder Smartphone ausgestaltet sein. Sinnvollerweise findet dabei die Datenübertragung über eine Funkstrecke statt, beispielsweise über eine Bluetooth-Verbindung.

### BEZUGSZEICHENLISTE

- 1: Ruderschaft
- 2: Ruderblatt
- 3: Messaufnahme
- 4: äusserer Dehnungsmessstreifen (DMS)
- 5: obere Klinge
- 7: Batterie
- 9: Rudergriff
- 10: ruderschaftseitige Hülse
- 11: rudergriffseitige Hülse
- 12: ruderschaftseitiger Aufnahmebereich
- 13: rudergriffseitiger Aufnahmebereich
- 14: innerer Dehnungsmessstreifen (DMS)
- 15: untere Klinge
- 16: S-förmig verformte Klinge
- 19: Flansch / Anschlag (für den Handgriff)
- 20: Innenteil
- 21: Aussengewinde des Innenteils
- 22: sich verjüngender hohler Kegelstumpfabschnitt
- 23: Endbereich der Hülse 20
- 24: Durchgangsloch
- 24': Fixierungsloch
- 25: PCB
- 29: Dichtungsring
- 50: Verarbeitungseinheit
- 81: Kurve des Biegemoments Steuerbord
- 82: Kurve des Biegemoments Backbord
- 100: Längsachse des Ruders (ohne Belastung)
- 100': Längsachse des Handgriffs (unter Belastung)
- 101: Zugrichtung

## Patentansprüche

1. System für ein Ruder zur Bestimmung der Ruderkraft während eines Ruderschlags eines Ruderers im Wasser aufweisend:
eine Messaufnahme (3) mit einem ruderschaftseitigen Aufnahmebereich (12) für eine Befestigung mit einem Ruderschaft (1) eines Ruders und mit einem rudergriffseitigen Aufnahmebereich (13) für eine Befestigung mit einem Rudergriff (9) diesen Ruders;
mindestens ein in der Messaufnahme (3) angeordneter Dehnungsmessstreifen (4, 14), aus dessen Signal sich eine Kraft bestimmen lässt; und
eine mit dem oder den Dehnungsmessstreifen (4, 14) verbundene Auswerteschaltung;
**dadurch gekennzeichnet, dass** die Längsachse (100) der Befestigung der Messaufnahme (3) an dem Ruderschaft (1) mit der Längsachse der Befestigung der Messaufnahme (3) an dem Rudergriff (9) ohne Belastung zusammenfällt, dass die Messaufnahme (3) mindestens eine Platte (5, 15) umfasst, die sich zwischen dem ruderschaftseitigen Aufnahmebereich (12) und dem rudergriffseitigen Aufnahmebereich (13) erstreckt, so dass bei einer Kraftausübung (101) auf den rudergriffseitigen Aufnahmebereich (13) senkrecht zur Ebene der Platte (5, 15) die Platte eine S-Form annimmt, dass zwei Dehnungsmessstreifen (4, 14) vorgesehen sind, die nahe des ruderschaftseitigen Aufnahmebereichs (12) oder nahe des rudergriffseitigen Aufnahmebereichs (13) auf beiden Seiten der Platte (5, 15) aufgebracht sind, und dass die Auswerteschaltung ausgestaltet ist, um aus der Dehnung und Stauchung der beiden Dehnungsmessstreifen (4, 14) die auf den Handgriff ausgeübte Kraft zu berechnen.

2. System nach Anspruch 1, wobei zwei Platten (5, 15) vorgesehen sind, die in einem radialen Abstand von der Längsachse (100) des ruderschaftseitigen Aufnahmebereichs (12) zur Befestigung der Messaufnahme (3) in Richtung der Kraftausübung (101) parallel zueinander vorgesehen sind.

3. System nach Anspruch 2, wobei die beiden Platten (5, 15) in einem gleichen radialen Abstand von der Längsachse (100) gegenüber angeordnet sind.

4. System nach Anspruch 3, wobei ein Innenteil (20) vorgesehen ist, welches in der Messaufnahme (3) zwischen den zwei Platten (5, 15) angeordnet ist, und welches Innenteil an dem ruderschaftseitigen Aufnahmebereichs (12) befestigt ist.

5. System nach Anspruch 4, wobei das Innenteil (20) ein Aussengewinde (21) aufweist, welches in ein Innengewinde des ruderschaftseitigen Aufnahmebereichs (12) einschraubbar ist, wobei das Innenteil (20) bis in den Handgriff (9) hineinreicht.

6. System nach Anspruch 5, wobei das Innenteil (20) in Richtung Handgriff (9) eine sich verjüngende hohle Kegelstumpfform zur führenden Aufnahme des Ruderschaftes (1) aufweist.

7. System nach einem der vorstehenden Ansprüche, dass ein mikroelektromechanisches Systems derart angeordnet ist, um die Rotationsbewegung des Systems um eine Achse senkrecht sowohl zur Längsachse (100) des ruderschaftseitigen Aufnahmebereichs (12) als auch senkrecht zur Ebene der Platte(n) (5, 15) oder Richtung der Kraftausübung (101) über die Zeit zu ermitteln, und dass die Auswerteschaltung ausgestaltet ist, den vorbestimmten Abstand der Drehachse von dem Handgriff zu erhalten, und weiter ausgestaltet ist, um die Ruderleistung über die Zeit aus dem skalaren Produkt der Kraft mit dem vorbestimmten Abstand der Drehachse von dem Handgriff und der Rotationsbewegung zu bestimmen.

8. System nach Anspruch 7, wobei die Werte der Rotationsbewegung und der erfassten Kraft an die Auswerteschaltung übermittelt werden, die sich in einem extern zu dem Ruder befindlichen Gerät befindet.

9. Ruder zur Bestimmung der Ruderkraft während eines Ruderschlags eines Ruderers im Wasser aufweisend:
einen Rudergriff (9);
einen Ruderschaft (1), welcher sich vom Rudergriff (9) weg erstreckt;
ein System nach einem der vorstehenden Ansprüche.
